# EUROPEAN PATENT APPLICATION

(11) **EP 4 768 473 A1**
(43) Date of publication of application: **01.07.2026**
(21) Application number: 24938104.7
(22) Date of filing: 15.10.2024
(51) Int. Cl.: C07D 249/12

(54) **1-HYDROXYALKYL-5-MERCAPTO-1,2,4-TRIAZOLE-3-CARBOXYLIC ACID COMPOUNDS, PREPARATION METHOD THEREFOR, AND USE THEREOF**

(71) Applicant: Max (Rudong) Chemicals Co., Ltd., Jiangsu 226400 (CN)
(72) Inventor: CHEN, Bangchi, Nantong, Jiangsu 226407 (CN); WANG, Junliang, Nantong, Jiangsu 226407 (CN); ZHOU, Junjin, Nantong, Jiangsu 226407 (CN); ZHANG, Zhiming, Nantong, Jiangsu 226407 (CN); DU, Xiaodong, Nantong, Jiangsu 226407 (CN); CHEN, Kaiquan, Nantong, Jiangsu 226407 (CN)
(74) Representative: Haseltine Lake Kempner LLP
(86) International application number: PCT/CN2024/124981
(87) International publication number: WO 2026/081073

(57) **Abstract**

A 1-hydroxyalkyl-5-mercapto-1,2,4-triazole-3-carboxylic acid compound of formula (1): 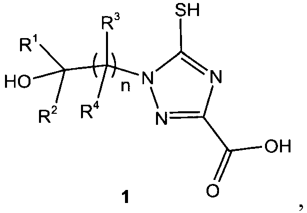 in which R¹, R², R³ and R⁴ are each independently selected from the group consisting of hydrogen, unsubstituted and substituted C₁-C₁₈ alkyl, unsubstituted and substituted C₁-C₁₈ alkenyl, unsubstituted and substituted C₃-C₁₈ cycloalkyl, unsubstituted and substituted C₆-C₁₄ aryl, unsubstituted and substituted C₁-C₁₃ heteroaryl, unsubstituted and substituted C₇-C₁₈ aralkyl, unsubstituted and substituted C₈-C₁₈ arylalkenyl, and unsubstituted and substituted C₇-C₁₈ aryloxyalkyl, and n is 1, 2, 3 or 4. A preparation method for the 1-hydroxyalkyl-5-mercapto-1,2,4-triazole-3-carboxylic acid compound is also provided, in which a hydroxyalkylhydrazine compound is used as a starting material, and undergoes a condensation reaction, an addition-cyclization reaction and dehydrogenation in sequence to obtain the 1-hydroxyalkyl-5-mercapto-1,2,4-triazole-3-carboxylic acid compound. An use of such compound in the preparation of a 2-hydroxyalkyl-1,2,4-triazole-3-thione compound is also provided.

## Description

### TECHNICAL FIELD

This application relates to organic synthesis, and more particularly to a class of 1-hydroxyalkyl-5-mercapto-1,2,4-triazole-3-carboxylic acid compounds, and preparation method and use thereof.

### BACKGROUND

Provided herein is a 1-hydroxyalkyl-5-mercapto-1,2,4-triazole-3-carboxylic acid compound of formula (1), represented by:

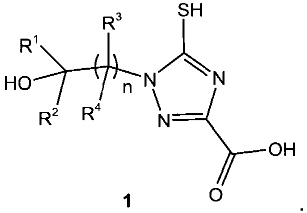

These structurally-novel compounds (1) which have not been reported are multifunctional substances. Through further functional group transformations, the compounds (1) are expected to be useful in syntheses of a variety of derivatives with different chemical property, physical property and biological activity.

For example, the compounds (1) can be applied to the preparation of certain practically-valuable products, such as the broad-spectrum fungicide 2-[2-(1-chlorocyclopropyl)-3-(2-chlorophenyl)-2-hydroxypropyl]-1,2,4-triazole-3-thione (Prothioconazole).

CN105949137 provides a detailed analysis and comparison of several known technology for the preparation of Prothioconazole, and discloses an improved synthetic method, which successfully addressed various issues in the previous methods by strategy of preparing 2-{2-[2-(1-chlorocyclopropyl)-3-(2-chlorophenyl)-2-hydroxypropyl]hydrazono}acetic acid as an intermediate. However, this intermediate compound suffers from product isolation problems in large scale production due to the presence of *cis*-*trans* isomerism associated with the hydrazinylidene C=N double bond.

The practical fungicidal applications of Prothioconazole have been disclosed in Patent Publications WO96/16048, WO99/18086, WO 99/18087 and WO 99/18088.

### SUMMARY

Technical solutions of the present disclosure are described as follows.

In a first aspect, the disclosure provides a 1-hydroxyalkyl-5-mercapto-1,2,4-triazole-3-carboxylic acid compound of formula (1), represented by: 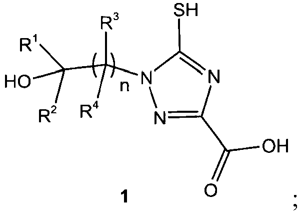
wherein R¹, R², R³ and R⁴ are each independently selected from the group consisting of hydrogen, unsubstituted and substituted C₁-C₁₈ alkyl, unsubstituted and substituted C₁-C₁₈ alkenyl, unsubstituted and substituted C₃-C₁₈ cycloalkyl, unsubstituted and substituted C₆-C₁₄ aryl, unsubstituted and substituted C₁-C₁₃ heteroaryl, unsubstituted and substituted C₇-C₁₈ aralkyl, unsubstituted and substituted C₈-C₁₈ arylalkenyl, and unsubstituted and substituted C₇-C₁₈ aryloxyalkyl; and
n is an integer selected from 1-4, such as 1, 2, 3 and 4.

In some embodiments, R¹, R², R³ and R⁴ are each independently selected from the group consisting of hydrogen, unsubstituted and substituted C₁-C₁₈ alkyl, unsubstituted and substituted C₃-C₁₈ cycloalkyl and unsubstituted and substituted C₇-C₁₈ aralkyl; and n is 1 or 2.

In some embodiments, R¹ is hydrogen, or unsubstituted or substituted C₇ aralkyl; R² is hydrogen, or unsubstituted and substituted C₃ cycloalkyl; R³ and R⁴ are hydrogen; and n is 1.

In some embodiments, R¹ is 2-chlorobenzyl, R² is 1-chlorocyclopropyl, R³ and R⁴ are hydrogen, and n is 1; or R¹, R², R³ and R⁴ are hydrogen, and n is 1.

In a second aspect, the disclosure provides a method for preparing the 1-hydroxyalkyl-5-mercapto-1,2,4-triazole-3-carboxylic acid compound (1) provided herein, comprising:
(a) subjecting a hydroxyalkylhydrazine compound (2) to a condensation reaction with glyoxylic acid or a glyoxylate salt to produce a condensation product;
(b) subjecting the condensation product to an addition-cyclization reaction with thiocyanic acid or a thiocyanate salt in the presence of an acid A to produce an addition-cyclization product; and
(c) subjecting the addition-cyclization product to dehydrogenation under the action of an oxidant to yield the 1-hydroxyalkyl-5-mercapto-1,2,4-triazole-3-carboxylic acid compound of formula (1), as represented by the following reaction scheme: 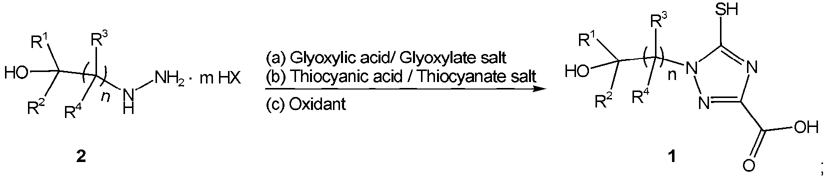 wherein R¹, R², R³ and R⁴ are each independently selected from the group consisting of hydrogen, unsubstituted and substituted C₁-C₁₈ alkyl, unsubstituted and substituted C₁-C₁₈ alkenyl, unsubstituted and substituted C₃-C₁₈ cycloalkyl, unsubstituted and substituted C₆-C₁₄ aryl, unsubstituted and substituted C₁-C₁₃ heteroaryl, unsubstituted and substituted C₇-C₁₈ aralkyl, unsubstituted and substituted C₈-C₁₈ arylalkenyl , and unsubstituted and substituted C₇-C₁₈ aryloxyalkyl; and n is an integer selected from 1-4, such as 1, 2, 3, 4.

In some embodiments, R¹, R², R³ and R⁴ are each independently selected from the group consisting of hydrogen, unsubstituted and substituted C₁-C₁₈ alkyl, unsubstituted and substituted C₃-C₁₈ cycloalkyl and unsubstituted and substituted C₇-C₁₈ aralkyl; and n is 1 or 2.

In some embodiments, R¹ is hydrogen, or unsubstituted and substituted C₇ aralkyl; R² is hydrogen, or unsubstituted and substituted C₃ cycloalkyl; R³ and R⁴ are hydrogen; and n is 1.

In some embodiments, R¹ is 2-chlorobenzyl, R² is 1-chlorocyclopropyl, R³ and R⁴ are hydrogen, and n is 1; or R¹ , R² , R³ and R⁴ are hydrogen and n is 1.

In some embodiments, m is 0-2, including 0, 0.5, 1, 1.5, 2, and any value therebetween.

In some embodiments, HX is selected from the group consisting of a hydrohalic acid, sulfuric acid and phosphoric acid; and the hydrohalic acid is hydrochloric acid or hydrobromic acid. In some embodiments, the hydrohalic acid is the hydrochloric acid.

In some embodiments, the glyoxylic acid is glyoxylic acid or a hydrate thereof; the glyoxylate salt is an alkali metal glyoxylate or ammonium glyoxylate; an alkali metal is selected from the group consisting of lithium, sodium and potassium; an ammonium is represented by NR⁵R⁶R⁷R⁸, wherein R⁵, R⁶, R⁷ and R⁸ are each independently selected from the group consisting of hydrogen, unsubstituted and substituted alkyl, unsubstituted and substituted aralkyl and unsubstituted and substituted aryl. In some embodiments, R⁵, R⁶, R⁷ and R⁸ are methyl, n-butyl.

In some embodiments, the thiocyanate salt is selected from the group consisting of an alkali metal thiocyanate, an alkaline-earth metal thiocyanate and ammonium thiocyanate; an alkali metal is selected from the group consisting of lithium, sodium and potassium; an alkaline-earth metal is preferably magnesium or calcium; an ammonium is represented by NR⁵R⁶R⁷R⁸, wherein R⁵, R⁶, R⁷ and R⁸ are each independently selected from the group consisting of hydrogen, unsubstituted and substituted alkyl, unsubstituted and substituted aralkyl and unsubstituted and substituted aryl. In some embodiments, R⁵, R⁶, R⁷ and R⁸ are methyl, n-butyl.

In some embodiments, the acid A is a protic acid; the acid A is selected from the group consisting of a hydrohalic acid, sulfuric acid, phosphoric acid and a carboxylic acid; the carboxylic acid is an alkyl carboxylic acid or an aryl carboxylic acid; the alkyl carboxylic acid includes a substituted alkyl carboxylic acid; the aryl carboxylic acid includes a substituted aryl carboxylic acid. In some embodiments, the acid A is selected from the group consisting of hydrochloric acid, sulfuric acid, formic acid, acetic acid, trifluoroacetic acid and benzenesulfonic acid.

In some embodiments, the oxidant is selected from the group consisting of ferric chloride, hydrogen peroxide, nitric acid, air, oxygen and a combination thereof. In some embodiments, the oxidant is oxygen or hydrogen peroxide.

In some embodiments, the method for preparing the 1-hydroxyalkyl-5-mercapto-1,2,4-triazole-3-carboxylic acid compound (1) is carried out stepwise or in a "one-pot" manner.

In a third aspect, the disclosure also provides a method for preparing a 2-hydroxyalkyl-1,2,4-triazole-3-thione compound of formula (3), comprising:
subjecting the 1-hydroxyalkyl-5-mercapto-1,2,4-triazole-3-carboxylic acid compound (1) provided herein to a decarboxylation reaction in the presence of a catalyst to yield the 2-hydroxyalkyl-1,2,4-triazole-3-thione compound, as represented by the following reaction scheme: 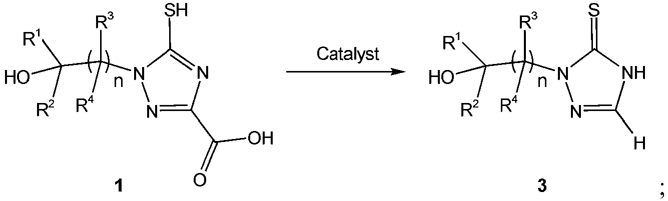
wherein R¹, R², R³ and R⁴ are each independently selected from the group consisting of hydrogen, unsubstituted and substituted C₁-C₁₈ alkyl, unsubstituted and substituted C₁-C₁₈ alkenyl, unsubstituted and substituted C₃-C₁₈ cycloalkyl, unsubstituted and substituted C₆-C₁₄ aryl, unsubstituted and substituted C₁-C₁₃ heteroaryl, unsubstituted and substituted C₇-C₁₈ aralkyl, unsubstituted and substituted C₈-C₁₈ aralkenyl, and unsubstituted and substituted C₇-C₁₈ aryloxyalkyl; and n is an integer selected from 1-4, such as 1, 2, 3, 4.

In some embodiments, R¹, R², R³ and R⁴ are each independently selected from the group consisting of hydrogen, unsubstituted and substituted C₁-C₁₈ alkyl, unsubstituted and substituted C₃-C₁₈ cycloalkyl and unsubstituted and substituted C₇-C₁₈ aralkyl; and n is 1 or 2.

In some embodiments, R¹ is hydrogen, or unsubstituted and substituted C₇ aralkyl; R² is hydrogen, or unsubstituted and substituted C₃ cycloalkyl; R³ and R⁴ are hydrogen; and n is 1.

In some embodiments, R¹ is 2-chlorobenzyl and R² is 1-chlorocyclopropyl; or R¹ and R² are hydrogen.

In some embodiments, the catalyst is an acid B; and the acid B is selected from the group consisting of a Brønsted acid, a Lewis acid and a combination thereof.

In some embodiments, the Brønsted acid is selected from the group consisting of sulfuric acid, hydrochloric acid, acetic acid and p-toluenesulfonic acid; and the Lewis acid is selected from the group consisting of ferric chloride, aluminum chloride and boron trifluoride. In some embodiments, the acid B is selected from the group consisting of hydrochloric acid, p-toluenesulfonic acid and ferric chloride.

In some embodiments, the decarboxylation reaction is carried out in a batch reactor or a continuous flow reactor.

In an embodiment, the 1-hydroxyalkyl-5-mercapto-1,2,4-triazole-3-carboxylic acid compound (where R¹ is 2-chlorobenzyl, R² is 1-chlorocyclopropyl, R³ and R⁴ are hydrogen and n is 1) can be further transformed to produce a broad-spectrum fungicide 2-[2-(1-chlorocyclopropyl)-3-(2-chlorophenyl)-2-hydroxypropyl]-1,2,4-triazole-3-thione (Prothioconazole).

Compared to the prior art, the present disclosure has the following beneficial effects.
1. The 1-hydroxyalkyl-5-mercapto-1,2,4-triazole-3-carboxylic acid compound (1) provided herein is structurally-novel and multi-functional.
2. The preparation of the 1-hydroxyalkyl-5-mercapto-1,2,4-triazole-3-carboxylic acid compound (1) has simple operation, high yield, low waste generation and excellent safety, making it suitable for the large-scale industrial production.
3. The 1-hydroxyalkyl-5-mercapto-1,2,4-triazole-3-carboxylic acid compound (1) can be applied to the synthesis of other valuable compounds through functional group transformation.
4. The 1-hydroxyalkyl-5-mercapto-1,2,4-triazole-3-carboxylic acid compound (1) has been successfully used in the preparation of the 2-[2-(1-chlorocyclopropyl)-3-(2-chlorophenyl)-2-hydroxypropyl]-1,2,4-triazole-3-thione (Prothioconazole), remedying many deficiencies in the existing preparation methods.

The compound described in the present disclosure is to be construed as including the compound itself, its stereoisomers, tautomers, isotopically labeled compounds, or agriculturally acceptable salts or esters thereof. The stereoisomers, tautomers, isotopically labeled compounds, or agriculturally acceptable salts of the compound can be obtained by conventional techniques known in the art and exert the same or similar effects through substantially the same mechanism of action, both in vivo and in vitro.

As used herein, the term "stereoisomer" refers to an isomer differing in the spatial arrangement of atoms within a molecule, including configurational isomers and conformational isomers. Configurational isomers further include geometric isomers (cis-trans isomers) and optical isomers (enantiomers and diastereomers). Geometric isomers may be present in the compounds of the present disclosure. Optical isomers refer to compounds with identical molecular structures and similar physicochemical properties but differing in optical activity. The compounds described herein may contain asymmetrically substituted carbon atoms in the R or S configuration (where the terms "R" and "S" are defined in IUPAC 1974 Recommendations for Section E, Fundamental Stereochemistry, Pure Appl. Chem. (1976) 45, 1310.). Compounds with asymmetrically substituted carbon atoms that have equal amounts of the R and S configurations are racemic at those carbon atoms. A compound with an excess of one configuration has a higher proportion of that configuration, preferably by about 85-90%, more preferably about 95-99%, and most preferably greater than 99%. Accordingly, the present disclosure includes racemic mixtures, individual relative or absolute optical isomers, and relative and absolute optical isomers mixtures.

As used herein, the term "tautomer" refers to structural isomers with different energy levels that can interconvert through a low-energy barrier. When tautomerism is possible (e.g., in solution), a chemical equilibrium between tautomers can be established. For example, proton tautomers (referred to as proton-transfer tautomers) include interconversions occurring via proton migration, such as keto-enol tautomerization and imine-enamine tautomerization. Valence tautomers involve reorganization of bonding electrons.

As used herein, the term "isotopically labeled derivative" refers to a compound of the present disclosure that exists in an isotopically labeled or enriched form, containing one or more atoms with atomic weights or mass numbers differing from those most abundantly found in nature. The isotopes may be either radioactive or nonradioactive. Isotopes of atoms including, but not limited to, hydrogen, carbon, nitrogen, oxygen, phosphorus, sulfur, fluorine, chlorine, bromine and iodine encompass, for example, ²H, ³H, ¹³C, ¹⁴C, ¹⁵N, ¹⁸O, ³²P, ³⁵S, ¹⁸F, ³⁶Cl, ⁸⁰Br, and ¹²⁵I. Compounds containing these and/or other isotopes of the aforementioned atoms fall within the scope of the present disclosure. The isotopically labeled compounds of the present disclosure may be prepared using conventional methods known to those skilled in the art.

As used herein, the term "agriculturally acceptable salt" refers to a salt formed by the reaction of the 1-hydroxyalkyl-5-mercapto-1,2,4-triazole-3-carboxylic acid compound with a chemically acceptable base. The chemically acceptable base may be an inorganic base (e.g., sodium hydroxide, potassium hydroxide, calcium hydroxide, sodium carbonate, potassium carbonate, sodium bicarbonate or potassium bicarbonate) or an organic base (e.g., trimethylamine and triethylamine). The agriculturally acceptable salt may also be a salt formed by the reaction of the 1-hydroxyalkyl-5-mercapto-1,2,4-triazole-3-carboxylic acid compound with a chemically acceptable acid. The chemically acceptable acid may be an inorganic acid (e.g., hydrochloric acid, sulfuric acid, phosphoric acid or hydrobromic acid) or an organic acid (e.g., oxalic acid, maleic acid, fumaric acid, malic acid, tartaric acid, citric acid or benzoic acid). Furthermore, the agriculturally acceptable salt may include potassium salts, sodium salts, ammonium salts, calcium salts, pyridinium salts, choline salts, hydrochloride salts, phosphate salts, acetate salts, benzenesulfonate salts, or oxalate salts.

As used herein, the term "agriculturally acceptable ester" refers to an ester formed by the reaction of a carboxyl group of the 1-hydroxyalkyl-5-mercapto-1,2,4-triazole-3-carboxylic acid compound provided herein with an alcohol, a thioester formed by the reaction of a mercapto group of the above compound with an acylating agent, or an ester formed by the reaction of a hydroxy group of the above compound with an acylating agent.

The term "halogen" as used herein refers to fluorine, chlorine, bromine and iodine.

As used herein, the term "unsubstituted or substituted" refers to substitution in which one or more hydrogen atoms on a carbon or nitrogen atom are independently selected from the group consisting of halogen, C₁-C₆ alkyl, C₁-C₆ haloalkyl, C₃-C₉ cycloalkyl, C₃-C₉ halocycloalkyl, C₂-C₆ alkenyl, C₂-C₆ haloalkenyl, C₂-C₆ alkynyl, C₂-C₆ haloalkynyl, C₁-C₆ alkoxy, C₁-C₆ haloalkoxy, C₁-C₆ alkylthio, C₁-C₆ haloalkylthio, C₁-C₆ alkylsulfinyl, C₁-C₆ haloalkylsulfinyl, C₁-C₆ alkylsulfonyl, C₁-C₆ haloalkylsulfonyl, C₂-C₇ alkylcarbonyl, C₂-C₇ haloalkylcarbonyl, C₂-C₇ alkylcarbonyloxy, C₂-C₇ haloalkylcarbonyloxy, C₁-C₆ alkylsulfonyloxy, C₁-C₆ haloalkylsulfonyloxy, C₂-C₇ alkoxycarbonyl, C₂-C₇ haloalkoxycarbonyl, C₂-C₇ alkylcarbonylamino, C₂-C₇ haloalkylcarbonylamino, C₂-C₇ alkoxycarbonylamino, C₂-C₇ haloalkoxycarbonylamino, C₂-C₇ alkylaminocarbonyl, C₂-C₇haloalkylaminocarbonyl, C₁-C₆ alkylamino, C₁-C₆ haloalkylamino, C₂-C₆ alkenylamino, C₂-C₆ haloalkenylamino, C₂-C₆ alkynylamino, C₂-C₆ haloalkynylamino, C₃-C₉ cycloalkylamino, C₃-C₉ halocycloalkylamino, C₃-C₇ alkenylaminocarbonyl, C₃-C₇ haloalkenylaminocarbonyl, C₃-C₇ alkynylaminocarbonyl, C₃-C₇ haloalkynylaminocarbonyl, C₄-C₁₀ cycloalkylaminocarbonyl, C₄-C₁₀ halocycloalkylaminocarbonyl, amino, carbamoyl, sulfamoyl, cyano, nitro, hydroxyl, carboxyl, optionally substituted phenyl, optionally substituted heterocyclyl, optionally substituted benzyl, optionally substituted phenylcarbonyl, and optionally substituted phenylamino.

As used herein, the term "alkyl" includes both straight-chain and branched alkyl groups, such as methyl, ethyl, propyl, butyl, isopropyl and tert-butyl.

As used herein, the term "cycloalkyl" refers to C₃-C₈ carbocyclic alkyl and heterocyclic alkyl groups. Examples of carbocyclic alkyl groups include cyclopropyl, cyclobutyl and cyclopentyl. Examples of heterocyclic alkyl groups include glycidyl, tetrahydrofuranyl, dioxanyl and pyrrolidinyl.

As used herein, the term "aryl" includes phenyl, naphthyl, anthryl and phenanthryl.

As used herein, the term "heteroaryl" refers to a group containing at least one aromatic ring, where one or more carbon atoms in the aromatic ring are substituted by heteroatoms selected from the group consisting of nitrogen, oxygen and sulfur. The heteroaryl groups include, but are not limited to, pyrrolyl, furyl, thienyl, imidazolyl, oxazolyl, thiazolyl, triazolyl, tetrazolyl, pyrazolyl, pyridyl, pyrazinyl, pyridazinyl, pyrimidinyl, and fused-ring systems such as indolyl, quinolinyl, carbazolyl and purinyl.

As used herein, the term "aralkyl" refers to an alkyl group in which one or more hydrogen atoms are substituted by an aryl group, such as a benzyl group.

As used herein, the term "arylalkenyl" refers to an alkenyl group in which one or more hydrogen atoms are substituted by an aryl group, such as styryl.

As used herein, the term "aryloxyalkyl" refers to an alkyl group in which one or more hydrogen atoms are substituted by an aryloxy group. An example of an aryloxyalkyl group is phenoxymethyl.

The term "alkali metal" refers to lithium (Li), sodium (Na), potassium (K), rubidium (Rb), cesium (Cs) and francium (Fr). The term "alkaline-earth metal" refers to beryllium (Be), magnesium (Mg), calcium (Ca), strontium (Sr), barium (Ba) and radium (Ra).

It should be noted that the definition of the number of carbon atoms in a carbon chain as used herein does not include carbon atoms in substituents. For example, the term "unsubstituted or substituted C₆-C₁₄ aryl" as used herein also falls within the scope of the present disclosure, even in cases where the total number of carbon atoms exceeds 14 due to the presence of substituents containing multiple carbon atoms on the aryl ring.

### DETAILED DESCRIPTION OF EMBODIMENTS

The technical solutions of the present disclosure will be described clearly and completely below. It is obvious that described herein are merely some embodiments of the present disclosure, rather than all embodiments of the present disclosure. Based on the embodiments in the present disclosure, all other embodiments obtained by those of ordinary skill in the art without making creative effort shall fall within the scope of the present disclosure defined by the appended claims.

A continuous flow reaction apparatus used in Example 7 is a silicon carbide microchannel reactor JD-A-20 manufactured by Jinde New Material Co., Ltd. The silicon carbide microchannel reactor consists of six identical modules connected in series, each having a liquid holdup of 20 mL. More specifically, the first to fifth modules serve as reaction modules with a total holdup of 100 mL and a temperature of 150°C. A sixth module is configured as a cooling module with a temperature of 45°C. An outlet of the sixth module is sequentially connected to a back pressure valve and a collection vessel.

### EXAMPLE 1

### Synthesis of 1-[2-(1-chlorocyclopropyl)-3-(2-chlorophenyl)-2-hydroxypropyl] -5-mercapto-1,2,4-triazole-3-carboxylic acid

To a 250 mL reaction flask were added 13.7 g of 2-(1-chlorocyclopropyl)-1-(2-chlorophenyl)-3-hydrazinylpropan-2-ol and 100 mL of acetonitrile, to which 4 g of a 50% aqueous glyoxylic acid solution was dropwise added. The reaction mixture was stirred at room temperature for 2 h, added with 50 mL of water and 4.6 g of ammonium thiocyanate, and dropwise added with 8 g of hydrochloric acid. The reaction mixture was stirred at room temperature for 6 h, and then left to stand for phase separation. The organic phase was cooled to 0°C, dropwise added with 15 g of hydrogen peroxide, and reacted at 0°C for 5 h. The reaction mixture was subjected to phase separation, and the organic phase was subjected to concentration, addition of toluene, water washing and solvent removal to afford 18.8 g of a solid product (97% yield). ¹H NMR(δ, DMSO-d6): 14.518 (m, 1H), 7.584-7.565 (m, 1H), 7.410-7.391 (m, 1H), 7.276-7.245 (m, 2H), 5.068 (s, 1H), 4.657-4.628 (d, 1H), 4.473-4.444 (d, 1H), 3.385 (s, 1H), 3.314-3.244 (m, 2H), 1.014-0.988, 0.827-0.794, 0.755-0.699 (m, 4H); MS: m/z=387.9([M+1]⁺).

### EXAMPLE 2

### Synthesis of 1-[2-(1-chlorocyclopropyl)-3-(2-chlorophenyl)-2-hydroxypropyl]-5-mercapto-1,2,4-triazole-3-carboxylic acid

To a 250 mL reaction flask were added 15.6 g of 2-(1-chlorocyclopropyl)-1-(2-chlorophenyl)-3-hydrazinylpropan-2-ol hydrochloride, 100 mL of acetic acid and 20 mL of water, to which 11.5 g of a 50% aqueous sodium glyoxylic acid solution was dropwise added. The reaction mixture was stirred at room temperature for 3 h, added with 20 g of water and 5.7 g of sodium thiocyanate, and dropwise added with 7.5 g of concentrated sulfuric acid. The reaction mixture was stirred at room temperature for 8 h, and then left to stand for phase separation. The organic phase was cooled to -5°C, and then reacted at -5°C under an air atmosphere for 7 h. The reaction mixture was subjected to phase separation, and the organic phase was subjected to concentration, addition of toluene, water washing and solvent removal to afford 19 g of a solid product (98% yield).

### EXAMPLE 3

### Synthesis of 1-[2-(1-chlorocyclopropyl)-3-(2-chlorophenyl)-2-hydroxypropyl] -5-mercapto-1,2,4-triazole-3-carboxylic acid

To a 250 mL reaction flask were added 23.4 g of 2-(1-chlorocyclopropyl)-1-(2-chlorophenyl)-3-hydrazinylpropan-2-ol hydrochloride, 120 mL of water and 20 mL of acetonitrile. The reaction mixture was reacted and then cooled to 10°C, dropwise added with 60 g of a 10% aqueous glyoxylic acid solution, and then stirred at 10°C for 3 h. The reaction mixture was filtered and washed with water to obtain a washed filter residue. To a 250 mL reaction flask were added the washed filter residue, 150 mL of acetic acid and 8.5 g of sodium thiocyanate, to which 12.6 g of sodium bisulfate was dropwise added. The reaction mixture was stirred at room temperature for 10 h, and then left to stand for phase separation. The organic phase was cooled to -10°C and reacted at -10°C under an oxygen atmosphere for 10 h. The reaction mixture was subjected to phase separation, and the organic phase was subjected to concentration, addition of toluene, water washing and solvent removal to afford 25.5 g of a solid product (98.5% yield).

### EXAMPLE 4

### Synthesis of 1-(2-hydroxyethyl)-5-mercapto-1,2,4-triazole-3-carboxylic acid

To a 500 mL reaction flask were added 15.7 g of 2-hydrazinoethanol and 100 mL of acetonitrile, to which 22.1 g of glyoxylic acid monohydrate was added. The reaction mixture was stirred at room temperature for 1 h, added with 100 mL of water and 25.3 g of potassium thiocyanate, and dropwise added with 31.6 g of hydrochloric acid. The reaction mixture was stirred at room temperature for 8 h, cooled to -10°C, portion-wise added with 59 g of ferric chloride hexahydrate, and reacted at -10°C for 5 h. The reaction mixture was subjected to phase separation, concentration, addition of toluene, water washing and solvent removal to afford 35.9 g of a solid product (95% yield). ¹H NMR(δ, DMSO-d6): 14.247 (s, 1H), 4.704 (s, 1H), 4.167-4.144 (t, 2H), 3.761-3.737 (t, 2H); MS: m/z=189.9([M+1]⁺).

### EXAMPLE 5

### Synthesis of 2-[2-(1-chlorocyclopropyl)-3-(2-chlorophenyl)-2-hydroxypropyl] -1,2,4-triazole-3-thione

To a 250 mL hydrothermal reactor were added 20 g of 1-[2-(1-chlorocyclopropyl)-3-(2-chlorophenyl)-2-hydroxypropyl]-5-mercapto-1,2,4-triazole-3-carboxylic acid and 100 mL of toluene, to which 0.5 g of p-toluenesulfonic acid was added. The reaction mixture was heated to 130°C, reacted at 130°C for 3 h, cooled, washed with water and evaporated to remove solvent to afford 17.6 g of a solid product (99% yield). ¹H NMR (δ, CDCl₃): 12.300 (s, 1H), 7.856 (s, 1H), 7.549-7.544, 7.534-7.530 (dd, 1H), 7.377-7.374, 7.362-7.358 (dd, 1H), 7.242-7.183 (m, 2H), 4.802-4.773 (d, 1H), 4.510-4.481 (d, 1H), 4.212 (s, 1H), 3.621-3.594 (d, 1H), 3.193-3.166 (d, 1H), 0.943-0.922 (m, 1H), 0.885-0.767 (m, 3H); MS: m/z=343.9 ([M+1]⁺).

### EXAMPLE 6

### Synthesis of 2-[2-(1-chlorocyclopropyl)-3-(2-chlorophenyl)-2-hydroxypropyl]-1,2,4-triazole-3-thione

To a 250 mL reaction flask were added 30 g of 1-[2-(1-chlorocyclopropyl)-3-(2-chlorophenyl)-2-hydroxypropyl]-5-mercapto-1,2,4-triazole-3-carboxylic acid and 100 mL of Hisol (No. 100 solvent oil), to which 0.5 g of a 10% FeCl₃ solution was dropwise added. The reaction mixture was heated to 150°C, reacted at 150°C for 2 h, cooled, washed with water and evaporated to remove solvent to afford 26.6 g of a solid product (99% yield).

### EXAMPLE 7

### Synthesis of 2-[2-(1-chlorocyclopropyl)-3-(2-chlorophenyl)-2-hydroxypropyl] -1,2,4-triazole-3-thione

A 40% tert-butanol solution of 1-[2-(1-chlorocyclopropyl)-3-(2-chlorophenyl) -2-hydroxypropyl]-5-mercapto-1,2,4-triazole-3-carboxylic acid was adjusted to pH 2 with hydrochloric acid. The resulting solution was introduced into a first module of a microchannel reactor (manufactured by Jinde New Material Co., Ltd) at a flow rate of 50 mL/min using an infusion pump, while a 5% aqueous hydrochloric acid solution was simultaneously introduced into a third module at a flow rate of 0.5 mL/min. Then the reaction mixture was sequentially directed into a cooling module and a back pressure valve, and then collected in a collection vessel. The collected reaction mixture was concentrated, washed with toluene and water, and subjected to solvent removal to afford a solid product (98% yield).

### EXAMPLE 8

### Synthesis of 2-(2-hydroxyethyl)-1,2,4-triazole-3-thione

To a 250 mL reaction flask were added 35.9 g of 1-(2-hydroxyethyl)-5-mercapto-1,2,4-triazole-3-carboxylic acid and 100 mL of acetic acid. The reaction mixture was heated under reflux for 5 h, cooled, washed with water and evaporated to remove solvent to afford 23 g of a solid product (98% yield). ¹H NMR (δ, CDCl₃):12.208 (s, 1H), 7.946 (s, 1H), 4.377-4.354 (t, 2H), 3.866-3.842 (t, 2H); MS: m/z=145.9([M+1]⁺).

It should be noted that the above embodiments are only used to illustrate the technical solutions of the present disclosure, rather than limiting the disclosure. Although the present disclosure has been described in detail with reference to the above embodiments, those of ordinary skill in the art could still make various modifications and substitutions to the technical solutions recited in the above embodiments. It should be understood that such modifications or substitutions made without departing from the spirit of the disclosure shall fall within the scope of the present disclosure defined by the appended claims.

## Claims

1. A 1-hydroxyalkyl-5-mercapto-1,2,4-triazole-3-carboxylic acid compound of formula (1), represented by: 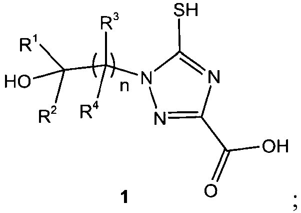
wherein R¹, R², R³ and R⁴ are each independently selected from the group consisting of hydrogen, unsubstituted and substituted C₁-C₁₈ alkyl, unsubstituted and substituted C₁-C₁₈ alkenyl, unsubstituted and substituted C₃-C₁₈ cycloalkyl, unsubstituted and substituted C₆-C₁₄ aryl, unsubstituted and substituted C₁-C₁₃ heteroaryl, unsubstituted and substituted C₇-C₁₈ aralkyl, unsubstituted and substituted C₈-C₁₈ arylalkenyl, and unsubstituted and substituted C₇-C₁₈ aryloxyalkyl; and
n is an integer selected from 1-4.

2. The 1-hydroxyalkyl-5-mercapto-1,2,4-triazole-3-carboxylic acid compound according to claim 1, **characterized in that** R¹, R², R³ and R⁴ are each independently selected from the group consisting of hydrogen, unsubstituted and substituted C₁-C₁₈ alkyl, unsubstituted and substituted C₃-C₁₈ cycloalkyl and unsubstituted and substituted C₇-C₁₈ aralkyl; n is 1 or 2; and preferably, R¹ is hydrogen, or unsubstituted and substituted C₇ aralkyl, R² is hydrogen, or unsubstituted and substituted C₃ cycloalkyl, R³ and R⁴ are hydrogen, and n is 1.

3. The 1-hydroxyalkyl-5-mercapto-1,2,4-triazole-3-carboxylic acid compound according to claim 2, characterized in thatR¹ is 2-chlorobenzyl, R² is 1-chlorocyclopropyl, R³ and R⁴ are hydrogen, and n is 1; or
R¹, R², R³ and R⁴ are hydrogen, and n is 1.

4. A method for preparing the 1-hydroxyalkyl-5-mercapto-1,2,4-triazole-3-carboxylic acid compound according to claim 1, comprising:
(a) subjecting a hydroxyalkylhydrazine compound (2) to a condensation reaction with glyoxylic acid or a glyoxylate salt to produce a condensation product;
(b) subjecting the condensation product to an addition-cyclization reaction with thiocyanic acid or a thiocyanate salt in the presence of an acid A to produce an addition-cyclization product; and
(c) subjecting the addition-cyclization product to dehydrogenation under the action of an oxidant to yield the 1-hydroxyalkyl-5-mercapto-1,2,4-triazole-3-carboxylic acid compound (1), as represented by the following reaction scheme: 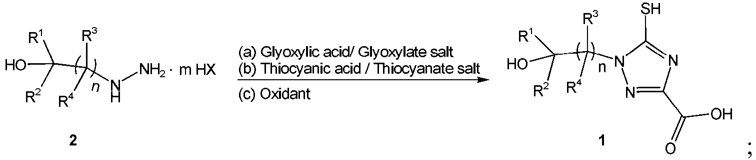
wherein R¹, R², R³, R⁴ and n are as defined in claim 1;
m is 0-2;
HX is selected from the group consisting of a hydrohalic acid, sulfuric acid and phosphoric acid; and
the acid A is a protic acid.

5. The method according to claim 4, **characterized in that** HX is a hydrohalic acid;
the glyoxylic acid is glyoxylic acid or a hydrate thereof;
the glyoxylate salt is an alkali metal glyoxylate or ammonium glyoxylate;
the thiocyanate salt is selected from the group consisting of an alkali metal thiocyanate, an alkaline-earth metal thiocyanate and ammonium thiocyanate;
the acid A is selected from the group consisting of a hydrohalic acid, sulfuric acid, phosphoric acid and a carboxylic acid;
the oxidant is selected from the group consisting of ferric chloride, hydrogen peroxide, nitric acid, air, oxygen and a combination thereof; and
m is 0 or 1.

6. The method according to claim 5, **characterized in that** HX is hydrochloric acid;
the glyoxylic acid is a glyoxylic acid hydrate;
the alkali metal glyoxylate is sodium glyoxylate;
the thiocyanate salt is an alkali metal thiocyanate, preferably sodium thiocyanate;
the acid A is selected from the group consisting of hydrochloric acid, sulfuric acid, formic acid, acetic acid, trifluoroacetic acid and benzenesulfonic acid; and
the oxidant is oxygen or hydrogen peroxide.

7. The method according to any one of claims 4-6, **characterized in that** the preparation of the compound (1) from the compound (2) is carried out stepwise or in a "one-pot" manner.

8. Use of the 1-hydroxyalkyl-5-mercapto-1,2,4-triazole-3-carboxylic acid compound according to claim 1, comprising:
subjecting the 1-hydroxyalkyl-5-mercapto-1,2,4-triazole-3-carboxylic acid compound to a decarboxylation reaction in the presence of a catalyst to yield a 2-hydroxyalkyl-1,2,4-triazole-3-thione compound of formula (3), as represented by the following reaction scheme: 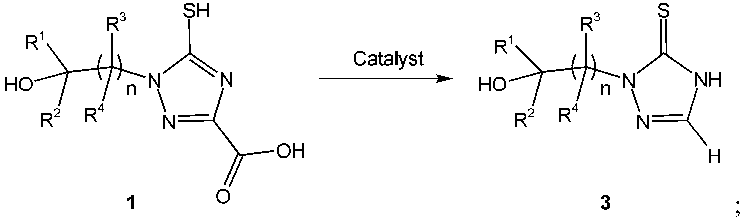
wherein R¹, R², R³, R⁴ and n are as defined in claim 1; and
the catalyst is an acid B.

9. The use according to claim 8, **characterized in that** the acid B is selected from the group consisting of a Brønsted acid, a Lewis acid and a combination thereof, preferably sulfuric acid, hydrochloric acid, acetic acid, p-toluenesulfonic acid, ferric chloride, aluminum chloride or boron trifluoride.

10. The use according to any one of claims 8-9, **characterized in that** the decarboxylation reaction is carried out in a batch reactor or a continuous flow reactor.
